# EUROPEAN PATENT SPECIFICATION

(11) **EP 4 166 091 B1**
(45) Date of publication and mention of the grant of the patent: **18.11.2026**
(21) Application number: 22197019.7
(22) Date of filing: 22.09.2022
(51) Int. Cl.: A61B 8/08, A61B 8/06, A61B 8/12, G01S 15/89

(54) **ULTRASOUND IMAGING OF CARDIAC ANATOMY USING DOPPLER ANALYSIS**
ULTRASCHALLBILDGEBUNG DER HERZANATOMIE MITTELS DOPPLERANALYSE
IMAGERIE ULTRASONORE DE L'ANATOMIE CARDIAQUE PAR ANALYSE DOPPLER

(30) Priority: 23.09.2021 US 202117483097
(43) Date of publication of application: 19.04.2023
(73) Proprietor: Biosense Webster (Israel) Ltd, 2066717 Yokneam (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(56) References cited:
- EP-A1- 3 682 810
- US-A- 5 622 174
- US-A1- 2013 303 907
- US-B1- 6 443 894

## Description

### FIELD OF THE INVENTION

The present invention relates generally to medical imaging, and particularly to Doppler ultrasound imaging using an intra-body medical ultrasound probe.

### BACKGROUND OF THE INVENTION

Ultrasound Doppler imaging techniques have been previously proposed in the art. For example, Sutherland describes noninvasive ultrasound Doppler myocardial imaging (DMI) in a paper titled, "Colour DMI: potential applications in acquired and congenital heart disease," ACTA PAEDIATRICA, Volume 84, Issue 410, August 1995, pages 40-48. The paper describes a DMI technique that allows colour Doppler imaging of cardiac structures as opposed to blood pool imaging. This is achieved by changing the velocity, filtering and threshold parameters of the standard colour Doppler algorithms. DMI parameters which can be measured are regional tissue velocity, acceleration and reflected Doppler energy. In addition, concomitant changes in the pulsed Doppler algorithms allow interrogation of instantaneous peak velocities during the cardiac cycle in the myocardial region in which the sample volume is placed.

Although the shape of specific elements of the heart, such as an ostium, may be reconstructed using known anatomical mapping methods, such methods typically rely on moving a catheter to touch points on the element. These approaches are computationally intensive and relatively time consuming. It would be useful to have a faster mapping method, and advantageous to have the method be non-contact.

EP3682810A1 describes an n intravascular ultrasound device comprising an array of ultrasound transducer elements which extends in a longitudinal and annular direction along and around the device body. US2013/303907A1 describes a device and system for imaging and blood flow velocity measurement. US5622174 describes an ultrasonic diagnosis apparatus and image displaying system. US6443894B1 describes a medical diagnostic ultrasound system and method for mapping surface data for three dimensional imaging.

### SUMMARY OF THE INVENTION

The invention is as defined in claim 1. Certain surgical methods are described with reference to the system of the present invention. Whilst no claim is directed to these methods per se, the system is capable of being used and is intended to be used in such methods.

There is provided, in accordance with an embodiment of the present invention, a system including a catheter including an array of ultrasound transducers and a processor. The array of ultrasound transducers is configured to be placed in a blood pool in an organ, to emit an ultrasound beam and to receive echo signals reflected in response to the ultrasound beam, to focus the emitted ultrasound beam at a given blood volume and receive echo signals reflected in response to the focused ultrasound beam, and to focus the emitted ultrasound beam by varying a focal length of the beam to variably collect blood Doppler shifted signals from different multiple blood volumes. The processor is configured to (a) distinguish, in the echo signals, between (i) first spectral signal components having Doppler shifts characteristic of blood and (ii) second spectral signal components having Doppler shifts characteristic of tissue of the organ, (b) calculate, based on the blood Doppler shifted signals collected from different multiple blood volumes, a blood velocity profile, (c) suppress the first spectral signal components relative to the second spectral signal components in the echo signals using the blood velocity profile, (d) reconstruct an ultrasound image of at least a portion of the organ from the echo signals having the suppressed first spectral signal components, and (e) display the reconstructed image to a user.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based ultrasound imaging system using a catheter with a distal end assembly comprising a 2D ultrasound-array and a location sensor, in accordance with an embodiment of the present invention;
Fig. 2 is a schematic, pictorial illustration of a process for isolation of a blood Doppler-shifted component from an echo signal, followed by reconstruction of a blood-signal-free image by the system of Fig. 1; and
Fig. 3 is a flow chart that schematically illustrates a method of isolation of blood Doppler-shifted components from an echo signal to generate a filtered image using the system of Fig. 1

### DETAILED DESCRIPTION OF EMBODIMENTS

### OVERVIEW

Embodiments of the present invention that are described hereinafter provide systems that use a probe, such as a catheter, having a two-dimensional (2D) array of ultrasound transducers, for producing three-dimensional (3D) or four-dimensional (4D) ultrasound images. In the present context, the term "3D ultrasound image" refers to an ultrasound image that represents a certain volume in three dimensions. The term "4D ultrasound image" refers to a time series of 3D ultrasound images of a certain volume. A 4D image can be regarded as a 3D movie, the fourth dimension being time. Another way of describing a 4D image (or rendering) is as a time-dependent 3D image (or rendering).

The 2D array produces a 3D sector-shaped ultrasound beam occupying a defined solid angle; (such a beam is referred to herein as a "wedge," as opposed to a 1D array "fan"). The 2D array is thus able to image a 2D section of an inner wall of an organ, such as of a cardiac chamber.

In some embodiments, a 4D ultrasound catheter is placed in the blood stream in proximity to an element to be mapped, such as a wall of a cardiac chamber. Subsequently, a processor analyzes reflected signals (e.g., echoes) from the ultrasound wedge beam transmitted by the catheter. Typically, the element of the heart is moving as the heart beats, as is the blood flowing through the heart. Both movements create Doppler shifts in the frequencies of the signals received by the transducers, but the flow of the blood, typically of the order of m/s, is significantly faster than any movement of the element being mapped. As such, the frequency shifts (Doppler shifts) in the echoes from blood are significantly larger than the Doppler shifts in the echoes from cardiac wall tissue. For example, for an ultrasound frequency of 5MHz, the Doppler shift in echoes from blood is on the order of 5kHz, whereas the Doppler shift in echoes from cardiac wall tissue is on the order of1KHz.

The processor analyzes the Doppler-shifted signals to find the positions and velocities of elements being imaged by the transducers. Because of the velocity difference between the liquid blood stream and the soft but solid tissue element, the Doppler shift due to the blood can be easily isolated (e.g., identified), to distinguish the surface of the element being mapped. The processor suppresses the blood Doppler-shifted component of the signal. For example, the processor may digitally filter out the Doppler-shifted components to completely remove them, or attenuates the blood-related spectral components by at least a given amount (e.g., by 20dB). The resulting enhanced signals can then be used to reconstruct a blood-signal-free ultrasound image of the element.

In an embodiment, a processor receives echo signals reflected in response to ultrasound beam emitted from an array of ultrasound transducers in a catheter placed in a blood pool in an organ. The processor distinguishes, in the echo signals, between (i) first spectral signal components having Doppler shifts characteristic of blood and (ii) second spectral signal components having Doppler shifts characteristic of tissue of the organ. The processor suppresses the first spectral signal components relative to the second spectral signal components in the echo signals. Then, the processor reconstructs an ultrasound image of at least a portion of the organ from the echo signals having the suppressed first spectral signal components, and displays the reconstructed image to a user

The phases of the 2D array of transducers can be electronically adjusted to focus at least a portion of the US wedge transmitted by the array on a target volume in the organ, such as a blood volume. This focusing effect may be used to temporarily increase the quality of the signals reflected from blood and/or from the cardiac wall, so as to enhance the Doppler measurement described above.

In some embodiments, the catheter also comprises an integral location sensor, such as a magnetic position sensor, that is pre-registered with the 2D array. Because of the integral location sensor, the spatial coordinates of every voxel in the imaged section are known. The processor can use the position measurements, for example, to overlay the blood-signal-free ultrasound image on another image (ultrasound or otherwise) of at least a portion of the heart.

Further to this, the processor can use the position measurements to register multiple ultrasound image sections, acquired by the 2D ultrasound transducer array, with one another. The processor then produces a union of the multiple registered ultrasound image sections, to form a rendering of at least a portion of the organ, and presents the rendering to a user.

In an embodiment, the processor performs the registration of the multiple ultrasound image sections while compensating for movements of the probe itself, or by compensating for movements due to respiration. In another embodiment, the processor produces the union by stitching the multiple ultrasound image sections one to another.

The processor can also adjust the phases of the driving signals to electronically steer the wedge, so that the imaged element (e.g., ostium of a pulmonary vein) is centered in a display showing the element.

### SYSTEM DESCRIPTION

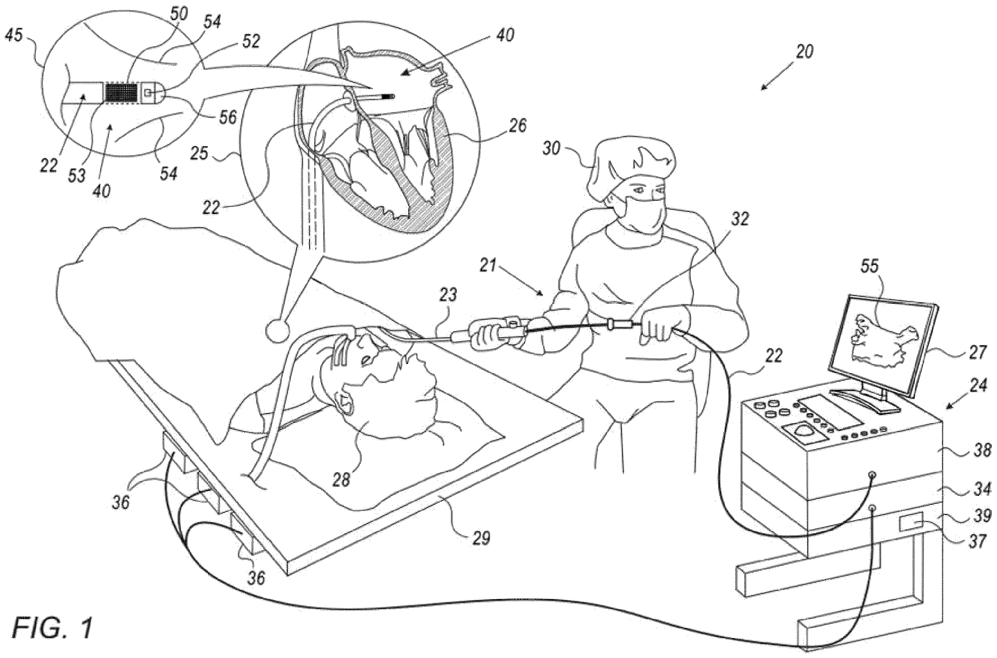

Fig. 1 is a schematic, pictorial illustration of a catheter-based ultrasound imaging system 20 using a catheter 21 with a distal end assembly 40 comprising a 2D ultrasound-array 50 and a location sensor 52, in accordance with an embodiment of the present invention. Integral location sensor 52 is pre-registered with the 2D array 50 of catheter 21.

As seen, distal end assembly 40 is fitted at the distal end of a shaft 22 of the catheter. Catheter 21 is inserted through a sheath 23 into a heart 26 of a patient 28 lying on a surgical table 29. The proximal end of catheter 21 is connected to a control console 24. In the embodiment described herein, catheter 21 is used for ultrasound-based diagnostic purposes, although the catheter may be further used to perform a therapy such as electrical sensing and/or ablation of tissue in heart 26, using, for example, a tip electrode 56.

Physician 30 navigates distal end assembly 40 of catheter 21 to a target location in heart 26 by manipulating shaft 22 using a manipulator 32 near the proximal end of the catheter.

In an embodiment, 2D ultrasound-array 50, shown in detail in an inset 25, is configured to image a left atrium of heart 26. The recorded images are stored by processor 30 in a memory 37.

As seen in an inset 45, ultrasound array 50 comprises a 2D array 50 of multiple ultrasound transducers 53. Inset 45 shows ultrasound array 50 navigated to an ostium wall 54 of a pulmonary vein of the left atrium. In this embodiment, 2D array 50 is an array of 32 × 64 US transducers. The 2D array is able to image a section of the inner wall of the ostium.

Sensor 52 is configured to output signals indicative of a position, direction and orientation of the 2D ultrasound transducer array 52 inside the organ. A processor of the system is configured to register multiple ultrasound image sections, one with the other, using the signal output by the sensor acquired by the 2D ultrasound transducer array 50.

Because of the integral location sensor, the spatial coordinates of every pixel in the imaged section are known.

Control console 24 comprises a processor 39, typically a general-purpose computer, with suitable front end and interface circuits 38 for driving ultrasound transducers 53 (e.g., in a phased array manner that includes steering an ultrasound beam), and for receiving echo signals from transducers 53 for use by processor 39. Interface circuits 38 are further used for receiving signals from catheter 21, as well as for, optionally, applying treatment via catheter 21 in heart 26 and for controlling the other components of system 20. Console 24 also comprises a driver circuit 34 configured to drive magnetic field generators 36.

During the navigation of distal end 22 in heart 26, console 24 receives position and direction signals from location sensor 52 in response to magnetic fields from external field generators 36. Magnetic field generators 36 are placed at known positions external to patient 28, e.g., below table 29 upon which the patient is lying. These position and direction signals are indicative of the position and direction of 2D ultrasound-array 50 in a coordinate system of the position tracking system.

The method of position and direction sensing using external magnetic fields is implemented in various medical applications, for example, in the CARTO^{™} system, produced by Biosense Webster, and is described in detail in U.S. Patents 6,618,612 and 6,332,089, in PCT Patent Publication WO 96/05768, and in U.S. Patent Application Publications 2002/0065455, 2003/0120150, and 2004/0068178.

In some embodiments, processor 39 may be configured to operate array 52 in an electronic "sweeping mode" to image a large portion of a cardiac camber. In an embodiment, the imaged cardiac chamber (e.g., a left atrium) is presented to physician 30 by processor 39 on a monitor 27, e.g., in as a volume rendering 55.

Processor 39 is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The example configuration shown in Fig. 1 is chosen purely for the sake of conceptual clarity. The disclosed techniques may similarly be applied using other system components and settings. For example, system 20 may comprise additional components and perform non-cardiac catheterizations.

### DOPPLER ANALYSIS IN 3D TO IMAGE HEART ANATOMY

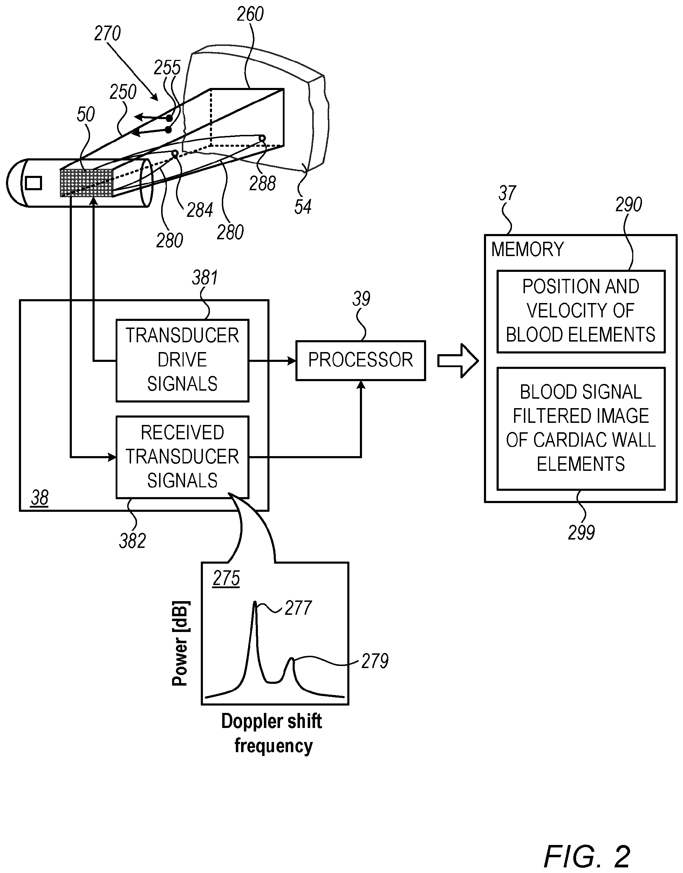

Fig. 2 is a schematic, pictorial illustration of a process for isolation of a blood Doppler-shifted component from an echo signal, followed by reconstruction of a blood-signal-free image 299 by system 20 of Fig. 1.

The ultrasound signals are transmitted in a form of a wedge beam 250, and echoes are detected by the same phased array 50 of catheter 21.

As seen, the mode of acquisition of 3D wedge beam 250 enables simultaneous acquisition of a 2D image section 260 of a surface of an ostium wall 54 over which blood 255 travel in a blood stream with a velocity 270, e.g., out of a pulmonary vein.

Processor 39 provides driving signals via a transducer driving unit 381 of interface circuits 38 for the ultrasound transducers. A receiving unit 382 of interface circuits 38 receives the echo signals from the transducers.

A schematic graph 275 shows an example of a spectrum of a resulting echo signal from a certain wedge. As seen, a blood Doppler-shifted component 279 is well resolved from a tissue Doppler-shifted component 277. Therefore, processor 39 can isolate and remove or attenuate blood peak 279, or otherwise take blood peak 279 into account in a reconstruction model, so as to image the geometry of section 260 with high accuracy. In particular, this technique enables the processor to accurately image the boundary between the blood and the cardia wall tissue.

In an embodiment, processor 39 thus analyzes the received signals (e.g., received echoes) from the transducers via receiving unit 382 of interface circuits 38 to:
(i) identify and filter out Doppler-shifted blood components from a signal, and, optionally, to derive position and velocity of blood elements 290; and
(ii) generate an image 299 of soft tissue (e.g., of cardiac wall surfaces) using the blood-signal filtered signals (i.e., echo signals without blood Doppler-shifted components)

Processor 39 saves the above information in memory 37.

The processor may filter out or attenuate Doppler-shifted blood component 279 using digital filtration applied in various different ways to digital echo signals that were digitized by unit 382. For example, in one embodiment, the processor may apply frequency-domain filtering that removes signal components having Doppler shifts corresponding to blood velocity, and retains signal components having Doppler shifts corresponding to cardiac-wall velocity. In another embodiment, the processor defines, in the frequency domain, one range corresponding to blood velocity and another (lower-frequency) range corresponding to cardiac-wall velocity. The processor then suppresses the spectral range related to blood. Note that for this kind of filtering, the processor may use, for example, simple threshold comparison and may not need to identify any spectral peaks in the signal.

In yet another embodiment, the processor identifies only the blood component in order to remove it, without identifying the wall-tissue component. In a further embodiment, the processor identifies the slow wall-related spectral component, and suppress other signals.

As noted above, processor 39 may adjust the relative phases of the driving signals provided to the 2D array of transducers to focus at least a portion 280 of ultrasound wedge beam 250 transmitted by the array onto a blood volume 284. This focusing effect may be used to enhance the Doppler measurement described above. Additionally or alternatively, for example at a slightly different time, the processor may focus portion 280 on wall tissue surface area 288, to further enhance the Doppler imaging technique.

To emit an ultrasound beam focused in a blood volume, the processor varies a focal length of the beam to collect a Doppler-shifted blood component from a location within this blood volume. By varying the locations, a blood velocity profile is characterized by the processor, for example, over a path in blood between the catheter and the wall surface. The resulting blood velocity profile is used in more elaborated (e.g., spatially weighted) removal of Doppler-shifted blood components of signals.

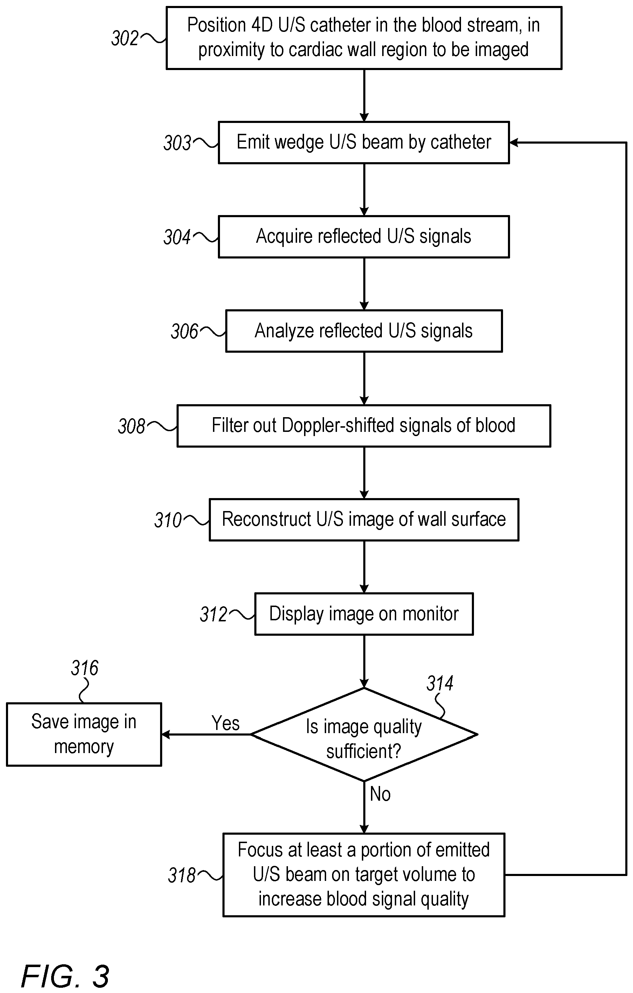

Fig. 3 is a flow chart that schematically illustrates a method of isolation of blood Doppler-shifted components from an echo signal to generate a filtered image 299 using system 20 of Fig. 1.

The process begins in positioning 4D ultrasound (US) catheter 21 in the blood stream in proximity to cardiac wall tissue region to be imaged, such as near ostium wall tissue 54 of Fig. 1, at a catheter placement step 302.

Next, processor 39 commands the emission of wedge ultrasound (U/S) beam 250 by catheter 21, by applying driving signals to 2D-array 50, using unit 381, at US emission step 303.

In a return signal acquisition step 304, a reflected US signal is acquired by processor 39 using array 50 and unit 382.

Processor 39 analyzes reflected US signals to identify different Doppler-shifted components (e.g., components 277 and 279 of Fig. 2) of the echo signals, at signal analysis step 306.

Then, processor 39 filters out blood Doppler-shifted components from reflected signals, at blood signal filtration out step 308.

Using the blood-signal filtered out signals, processor 39 reconstructs a US image, such as image 299, of ostium wall tissue 54, at a wall surface image reconstruction step 310.

At a displaying step 312, processor 39 displays the reconstructed image on monitor 27.

A reviewer, such as physician 30, can decide (314) if the blood-signal filtered US image is of sufficient quality, and save the image in memory 37, at image saving step 316.

If physician 30 deems (314) that the quality of the image is not good enough, then, based on his/her input, processor 39 focuses a portion of the emitted US beam on blood volume, at US beam focusing step 318, to obtain an improved acquisition of blood signal, and the process returns to step 303.

The flow chart of Fig. 3 is brought purely by way of example for the sake of conceptual clarity. For example, using blood signals to obtain blood information 290 is omitted for simplicity. As another example, the processor may additionally electronically steer wedge 250 during acquisition.

Although the embodiments described herein mainly address cardiac applications, the methods and systems described herein can also be used in other body organs. For example, the disclosed technique can be used with visualizing large blood vessels of the body, such as located in the abdomen and the brain.

It will thus be appreciated that the embodiments described above are cited by way of example, and that the present invention is not limited to what has been particularly shown and described hereinabove. Rather, the scope of the present invention is defined by claim 1 and may include both combinations and sub-combinations of the various features described hereinabove, as well as variations and modifications thereof which would occur to persons skilled in the art upon reading the foregoing description, provided the corresponding subject matter falls under the scope of the appended claims.

## Claims

1. A system (20), comprising:
a catheter comprising an array of ultrasound transducers, the array configured to be placed in a blood pool in an organ, to emit an ultrasound beam and to receive echo signals reflected in response to the ultrasound beam, wherein the array is configured to focus the emitted ultrasound beam at a given blood volume and receive echo signals reflected in response to the focused ultrasound beam, and wherein the array is configured to focus the emitted ultrasound beam by varying a focal length of the beam to variably collect blood Doppler shifted signals from different multiple blood volumes; and
a processor (39), which is configured to:
distinguish, in the echo signals, between (i) first spectral signal components (279) having Doppler shifts characteristic of blood (255) and (ii) second spectral signal components (277) having Doppler shifts characteristic of tissue (54) of the organ;
calculate, based on the blood Doppler shifted signals collected from different multiple blood volumes, a blood velocity profile;
suppress the first spectral signal components relative to the second spectral signal components in the echo signals using the blood velocity profile;
reconstruct an ultrasound image (299) of at least a portion of the organ from the echo signals having the suppressed first spectral signal components; and
display the reconstructed image to a user.

2. The system according to claim 1, wherein the processor is configured to suppress the first spectral signal components by filtering out the first spectral signal components from the echo signals.

3. The system according to claim 1, wherein the processor is configured to suppress the first spectral signal components by attenuating the first spectral signal components in the echo signals by at least a given amount.

4. The system according to claim 1, wherein the tissue of the organ is a wall tissue of a cardiac chamber.

## Patentansprüche

1. System (20), umfassend:
einen Katheter, umfassend eine Anordnung von Ultraschallwandlern, wobei die Anordnung konfiguriert ist, um in einem Blutpool in einem Organ platziert zu werden, um einen Ultraschallstrahl abzugeben und um Echosignale zu empfangen, die als Reaktion auf den Ultraschallstrahl reflektiert werden, wobei die Anordnung konfiguriert ist, um den abgegebenen Ultraschallstrahl auf ein vorgegebenes Blutvolumen zu fokussieren und Echosignale zu empfangen, die als Reaktion auf den fokussierten Ultraschallstrahl reflektiert werden, und wobei die Anordnung konfiguriert ist, um den abgegebenen Ultraschallstrahl durch Variieren einer Brennweite des Strahls, um Doppler-verschobene Blutsignale aus verschiedenen mehreren Blutvolumina variabel zu erfassen, zu fokussieren; und
einen Prozessor (39), der konfiguriert ist zum:
Unterscheiden, in den Echosignalen, zwischen (i) ersten Spektralsignalkomponenten (279), die für Blut (255) charakteristische Dopplerverschiebungen aufweisen, und (ii) zweiten Spektralsignalkomponenten (277), die für Gewebe (54) des Organs charakteristische Dopplerverschiebungen aufweisen;
Berechnen, basierend auf den Doppler-verschobenen Blutsignalen, die aus verschiedenen mehreren Blutvolumina erfasst wurden, eines Blutgeschwindigkeitsprofils;
Unterdrücken der ersten Spektralsignalkomponenten relativ zu den zweiten Spektralsignalkomponenten in den Echosignalen unter Verwendung des Blutgeschwindigkeitsprofils;
Rekonstruieren eines Ultraschallbildes (299) von mindestens einem Teil des Organs aus den Echosignalen, die die unterdrückten ersten Spektralsignalkomponenten aufweisen; und
Anzeigen des rekonstruierten Bildes an einen Benutzer.

2. System nach Anspruch 1, wobei der Prozessor konfiguriert ist, um die ersten Spektralsignalkomponenten durch Herausfiltern der ersten Spektralsignalkomponenten aus den Echosignalen zu unterdrücken.

3. System nach Anspruch 1, wobei der Prozessor konfiguriert ist, um die ersten Spektralsignalkomponenten durch Dämpfen der ersten Spektralsignalkomponenten in den Echosignalen um mindestens einen vorgegebenen Betrag zu unterdrücken.

4. System nach Anspruch 1, wobei das Gewebe des Organs ein Wandgewebe einer Herzkammer ist.

## Revendications

1. Système (20), comprenant :
un cathéter comprenant un réseau de transducteurs à ultrasons, le réseau étant configuré pour être placé dans un pool sanguin dans un organe, pour émettre un faisceau d'ultrasons et pour recevoir des signaux d'écho réfléchis en réponse au faisceau d'ultrasons, dans lequel le réseau est configuré pour focaliser le faisceau d'ultrasons émis au niveau d'un volume de sang donné et recevoir des signaux d'écho réfléchis en réponse au faisceau d'ultrasons focalisé, et dans lequel le réseau est configuré pour focaliser le faisceau d'ultrasons émis en faisant varier une longueur focale du faisceau pour collecter de manière variable des signaux sanguins décalés par effet Doppler à partir de multiples volumes de sang différents ; et
un processeur (39) qui est configuré pour :
distinguer, dans les signaux d'écho, entre (i) des premières composantes de signal spectral (279) ayant des décalages Doppler caractéristiques du sang (255) et (ii) des secondes composantes de signal spectral (277) ayant des décalages Doppler caractéristiques du tissu (54) de l'organe ;
calculer, sur la base des signaux sanguins décalés par effet Doppler recueillis à partir de multiples volumes sanguins différents, un profil de vitesse sanguine ;
supprimer les premières composantes de signal spectral par rapport aux secondes composantes de signal spectral dans les signaux d'écho en utilisant le profil de vitesse sanguine ;
reconstruire une image ultrasonore (299) d'au moins une partie de l'organe à partir des signaux d'écho ayant les premières composantes de signal spectral supprimées ; et
afficher l'image reconstruite à un utilisateur.

2. Système selon la revendication 1, dans lequel le processeur est configuré pour supprimer les premières composantes de signal spectral en éliminant par filtrage les premières composantes de signal spectral des signaux d'écho.

3. Système selon la revendication 1, dans lequel le processeur est configuré pour supprimer les premières composantes spectrales de signal en atténuant les premières composantes spectrales de signal dans les signaux d'écho d'au moins une quantité donnée.

4. Système selon la revendication 1, dans lequel le tissu de l'organe est un tissu de paroi d'une cavité cardiaque.
